(19) Europäisches Patentamt
European Patent Office
Office européen des brevets

(11) **EP 3 148 399 B1**

(12) **EUROPEAN PATENT SPECIFICATION**

(45) Date of publication and mention
of the grant of the patent:
**22.09.2021 Bulletin 2021/38**

(21) Application number: **15803734.1**

(22) Date of filing: **27.04.2015**

(51) Int Cl.:
*A61B 1/05* (2006.01)    *G03B 15/00* (2021.01)
*G06K 9/62* (2006.01)    *G06T 11/60* (2006.01)
*A61B 1/00* (2006.01)    *A61B 1/04* (2006.01)
*G06T 3/40* (2006.01)    *G06T 7/00* (2017.01)

(86) International application number:
**PCT/US2015/027813**

(87) International publication number:
**WO 2015/187261 (10.12.2015 Gazette 2015/49)**

(54) **RECONSTRUCTION OF IMAGES FROM AN IN VIVO MULTI-CAMERA CAPSULE WITH CONFIDENCE MATCHING**

REKONSTRUKTION VON BILDERN AUS EINER IN-VIVO-MULTIKAMERAKAPSEL MIT KONFIDENZABGLEICH

RECONSTITUTION D'IMAGES À PARTIR D'UNE CAPSULE IN VIVO À CAMÉRAS MULTIPLES AVEC MISE EN ÉQUIVALENCE DE LA CONFIANCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2014 US 201462006257 P**

(43) Date of publication of application:
**05.04.2017 Bulletin 2017/14**

(73) Proprietors:
- **Wang, Kang-Huai**
  **Saratoga, California 95070 (US)**
- **Wu, Chenyu**
  **Sunnyvale, CA 94087 (US)**
- **Capso Vision, Inc.**
  **Saratoga, CA 95070 (US)**

(72) Inventors:
- **WANG, Kang-Huai**
  **Saratoga, CA 95070 (US)**
- **WU, Chenyu**
  **Sunnyvale, CA 94087 (US)**

(74) Representative: **Papula Oy**
**P.O. Box 981**
**00101 Helsinki (FI)**

(56) References cited:
US-A- 6 044 181    US-A- 6 075 905
US-A1- 2009 074 265    US-A1- 2010 149 183
US-A1- 2011 261 262    US-A1- 2012 086 771
US-A1- 2012 154 562    US-A1- 2012 209 287

**Description**

**FIELD OF THE INVENTION**

The present invention relates to image stitching from images captured using in vivo capsule camera and their display thereof.

**BACKGROUND AND RELATED ART**

Capsule endoscope is an in vivo imaging device which addresses many of problems of traditional endoscopes. A camera is housed in a swallowable capsule along with a radio transmitter for transmitting data to a base-station receiver or transceiver. A data recorder outside the body may also be used to receive and record the transmitted data. The data primarily comprises images recorded by the digital camera. The capsule may also include a radio receiver for receiving instructions or other data from a base-station transmitter. Instead of using radio-frequency transmission, lower-frequency electromagnetic signals may be used. Power may be supplied inductively from an external inductor to an internal inductor within the capsule or from a battery within the capsule. In another type of capsule camera with on-board storage, the captured images are stored on-board instead of transmitted to an external device. The capsule with on-board storage is retrieved after the excretion of the capsule. The capsule with on-board storage provides the patient the comfort and freedom without wearing the data recorder or being restricted to proximity of a wireless data receiver.

While forward-looking capsule cameras include one camera, there are other types of capsule cameras that use multiple cameras to provide side view or panoramic view. A side or reverse angle is required in order to view the tissue surface properly. It is important for a physician or diagnostician to see all areas of these organs, as polyps or other irregularities need to be thoroughly observed for an accurate diagnosis. A camera configured to capture a panoramic image of an environment surrounding the camera is disclosed in US Patent Application, No. 11/642,275, entitled "In vivo sensor with panoramic camera" and filed on Dec. 19, 2006. A workstation-based, review and navigation system for in-vivo composite panoramic images is disclosed in US 2009/074265 A1, and systems and methods for performing image mosaicing are disclosed in US 2010/149183 A1

In an autonomous capsule system, multiple images along with other data are collected during the course when the capsule camera travels through the gastrointestinal (GI) tract. The images and data after being acquired and processed are usually displayed on a display device for a diagnostician or medical professional to examine. However, each image only provides a limited view of a small section of the GI tract. It is desirable to form a large picture from multiple capsule images representing a single composite view. For example, multiple capsule images may be used to form a cut-open view of the inner GI tract surface. The large picture can take advantage of the high-resolution large-screen display device to allow a user to visualize more information at the same time. The image stitching process may involve removing the redundant overlapped areas between images so that a larger area of the inner GI tract surface can be viewed at the same time as a single composite picture. In addition, the large picture can provide a complete view or a significant portion of the inner GI tract surface. It should be easier and faster for a diagnostician or a medical professional to quickly spot an area of interest, such as a polyp.

In the field of computational photography, image mosaicing techniques have been developed to stitch smaller images into a large picture. A review of general technical approaches to image alignment and stitching can be found in "Image Alignment and Stitching: A Tutorial", by Szeliski, Microsoft Research Technical Report MSR-TR-2004-92, December 10, 2006.

For image mosaicing, corresponding parts, objects or areas among images are identified first. After corresponding parts, objects or areas are identified, in other words, after two images are registered, they can be stitched by aligning the corresponding parts, objects or areas. Two images can be registered directly in the pixel domain or matched based on features extracted from images. The pixel-based image matching is also called direct match. There are several similarity measurements that can be used for evaluating the quality of image matching, such as sum of squared distance (SSD), normalized cross correlation (NCC), mutual information (MI) etc. NCC is equivalent to SSD if we apply normalization to SSD. Specifically, to match images from two different modalities, the mutual information of images A and B is defined as:

$$I(A,B) = \sum_{a,b} p(a,b)\log(\frac{p(a,b)}{p(a)p(b)}). \qquad (1)$$

The mutual information measures the distance between the joint distribution of the images intensity values $p(a,b)$ and the joint distribution of the images intensity values when they are independent, $p(a)p(b)$. It is a measure of dependence between two images. The assumption is that there is maximal dependence between the intensity values of the images when they are correctly aligned. Mis-registration will result in a decrease in the measure of mutual information. Therefore, larger mutual information implies more reliable registration.

The feature-based matching first determines a set of feature points in each image and then compares the corresponding feature descriptors. To match two image patches or features captured from two different viewing angles, a rigid model including scaling, rotation, etc. is estimated based on the correspondences. To match two images captured deforming objects, a non-rigid model including local deformation can be computed.

The number of feature points is usually much smaller than the number of pixels of a corresponding image. Therefore, the computational load for feature-based image matching is substantially less that for pixel-based image matching. However, it is still time consuming for pair-wise matching. Usually k-d tree, a well-known technique in this field, is utilized to speed up this procedure. Accordingly, feature-based image matching is widely used in the field. Nevertheless, the feature-based matching may not work well for images under some circumstances. In this case, the direct image matching can always be used as a fall back mode, or a combination of the above two approaches may be preferred.

Image matching techniques usually assume certain motion models. When the scenes captured by the camera consist of rigid objects, image matching based on either feature matching or pixel domain matching will work reasonably well. However, if the objects in the scene deform or lack of distinguishable features, it would make the image matching task very difficult. For capsule images captured during the course of travelling through the GI track, the situation is even more challenging. Not only the scenes corresponding to walls of the GI track deform while camera is moving and often are lack of distinguishable features, but also the scenes are captured with a close distance from the camera. Due to the close distance between objects and the camera, the often used linear camera model may fail to produce good match between different scenes. In addition, light reflection from near objects may cause over exposure for some parts of the object. Therefore, it is desirable to develop methods that can overcome the issues mentioned.

## SUMMARY OF INVENTION

A method of adaptively displaying images captured using a camera according to quality of image matching is disclosed. While image stitching provides an efficient viewing or examination of a large number of images, image stitching may cause noticeable artifacts particularly for images that do not fit camera models well. The present invention utilizes the quality of image matching to guide whether to stitch underlying images or not. Accordingly, an improved image reconstruction and a more visually pleasant viewing are achieved. In one embodiment, a plurality of images captured by the camera is received and the quality of image matching for each pair of images is determined. The quality of image matching is measured between two images to be matched. The image pair is declared as a high-confidence image pair or a low-confidence image pair according to the quality of image matching. The high-confidence image pairs are stitched into one or more larger composite pictures and displayed on a display device. On the other hand, the low-confidence image pairs are displayed as individual images without stitching.

The high-confidence matched images and the low-confidence unmatched images can be displayed on the display device in an interleaved manner, where the high-confidence matched images and the low-confidence unmatched images take turns to be displayed. The high-confidence matched images and the low-confidence unmatched images can also be displayed concurrently on the display device. In this case, the high-confidence matched images are displayed in one display area and the low-confidence unmatched images are displayed in another display area.

One aspect of the invention addresses various measurement of the quality of image matching. For example, the quality of image matching can be based on posterior probability corresponding to correct image matching given the features extracted and each matched feature pair is modelled as a binary random variable being an inlier or an outlier. In this case, the quality of image matching can be simply measured by counting the number of the matched feature pairs belonging to the inliers. The image pair is designated as a high-confidence matched image pair if the number of the features belonging to the inliers exceeds a threshold and, otherwise, the image pair is designated as a low-confidence unmatched image pair. The threshold is dependent on the probability of the feature being the inlier and the probability of the feature being the outlier.

The quality of image matching can also be based on pixel-domain measurement. For example, the quality of image matching can be based on the sum of squared distance (SSD) between two images to be matched. The image pair is designated as a high-confidence matched image pairs if the SSD is smaller than a threshold and, otherwise the image pair is designated as a low-confidence unmatched image pairs. Alternatively, the quality of image matching can be based on normalized cross correlation (NCC) or mutual information (MI) between two images to be matched. The image pair is designated as the high-confidence matched image pairs if the NCC or MI exceeds a threshold and, otherwise, the image pair is designated as the low-confidence unmatched image pairs.

For more complicated cases involving local deformation in addition to global transformation, the quality of image matching can be evaluated by combining image pyramids and free-form deformation such as B-spline deformation.

Searching for good global matching can start from coarse level of the image pyramid by down-sampling the original image. In each pyramid level, global transformation can be estimated by averaging local shifting or doing exhaustive search. Such a global transformation will be refined in the next level of the image pyramid until the final level which is the original image. This procedure can also be iterated to compensate the outlier effects. After the global transformation is estimated, free-form deformation can be estimated in the overlap area by dividing the overlap area into a set of control points/grids. The output of the optimization function can be used as a confidence score to categorize image pairs into high-confidence matched image pairs or low-confidence unmatched image pairs.

**BRIEF DESCRIPTION OF THE DRAWINGS**

Fig. 1 illustrates an exemplary high-confidence and low-confidence image pair's determination based on image matching, and displaying the high-confidence matched and unmatched low-confidence images in the same display area in an interleaved manner according to an embodiment of the present invention.

Fig. 2 illustrates an exemplary high-confidence and low-confidence image pair's determination based on image matching, and displaying the high-confidence matched and low-confidence unmatched images in respective display areas according to an embodiment of the present invention.

Fig. 3 illustrates an exemplary flowchart for a system for displaying images incorporating image stitching guided by quality of image matching according to an embodiment of the present invention.

**DETAILED DESCRIPTION OF THE INVENTION**

The following detailed description of the embodiments of the systems and methods of the present invention, as represented in the figures, is not intended to limit the scope of the invention as defined by the claims.

As mentioned before, image matching may not work well for images under some circumstances, particularly for images captured using a capsule image through the human gastrointestinal (GI) track. Embodiments according to the present invention use a quality measure of image matching. According to the measured matching quality, a matching confidence level is determined. When the matching confidence level is good enough, the underlying images are stitched. Otherwise, the underlying images are not stitched. For example, if feature based image matching is used, image matching will be performed to match many correspondences. After matching, RANdom Sample Consensus (RANSAC) process will be used to select a set of inliers that are compatible with a transformation model between the images. RANSAC is a well-known technique in the field that is used to find a best transform among feature points between two images. In order to verify the match, a confidence score is calculated based on a probabilistic model.

For each pair of matching images, a subset of feature correspondences that are geometrically consistent (i.e., RANSAC inliers), and the remaining features are not consistent (i.e., RANSAC outliers). To verify the set of correspondences, the probabilities that the set of features is generated by correct image matching (i.e., inliers) or by false image matching (i.e., outliers) are evaluated. For a given image, the total number of features is denoted as $n_f$ and the number of inliers is denoted as $n_i$, the event that image matching correctly/incorrectly is represented by a binary variable $m \in \{0,1\}$, where $m=1$ represents correct match and $m=0$ represents incorrect match. The event that the $i^{th}$ feature correspondence is an inlier/outlier, represented by the binary variable $f^{(i)} \in \{0,1\}$, where $f=1$ represents inlier and $f=0$ represents outlier, is assumed to be an independent Bernoulli distribution. Accordingly, the probability of all features being inliers is Binomial distribution:

$$p\big(f^{(1:n_f)}|m=1\big) = B\big(n_i; n_f, p_1\big), \text{ and} \tag{2}$$

$$p\big(f^{(1:n_f)}|m=0\big) = B\big(n_i; n_f, p_0\big), \tag{3}$$

where $p_1$ is the probability that a feature is an inlier given correct image matching, and $p_0$ is the probability that a feature is an inlier given false image matching. The total number of inliers, $n_i$ is calculated according to $n_i = \sum_{i=1}^{n_f} f^{(i)}$. The posterior probability that image matching is correct can be evaluated using Bayes's Rule:

$$p\left(m = 1 | f^{(1:n_f)}\right) = p\left(f^{(1:n_f)} | m = 1\right) p(m = 1) / p\left(f^{(1:n_f)}\right)$$

$$= 1/(1 + p\left(f^{(1:n_f)} | m = 0\right) p(m = 0) / p\left(f^{(1:n_f)} | m = 1\right) p(m = 1)) \quad (4)$$

Let the event of images matching correctly/incorrectly be a uniform prior (i.e., a prior probability distribution), p(m = 0) = p(m = 1). A criterion to accept image matching is based on whether $p\ (m = 1 | f^{(1:nf)}) > p_{min}$, where $p_{min}$ is a minimum probability threshold used as a criterion to accept the image matching. Through a sequence of mathematically derivation, this condition is reduced to a likelihood ratio test:

$$B\left(n_i; n_f, p_1\right) / B\left(n_i; n_f, p_0\right) \quad \begin{array}{c} accept \\ > \\ < \\ reject \end{array} \quad \frac{1}{1/p_{\min} - 1}, \text{ and} \quad (5)$$

$$B\left(n_i; n_f, p_1\right) = \binom{n_f}{n_i} p_1{}^{n_i} (1 - p_1)^{n_f - n_i} \quad (6)$$

The values for $p_{min}$, $p_1$ and $p_0$ can be properly chosen according to image models or based on test data. The above decision process can be further simplified as the following testing:

$$n_i > g\left(n_f\right), \quad (7)$$

where g is a function of $p_{min}$, $p_1$ and $p_0$. In other words, after the values for $p_{min}$, $p_1$ and $p_0$ are determined, *g* can be determined. The decision process simply becomes counting the number of inlier, $n_i$. If the condition of eqn. (7) is satisfied, the image matching is verified and the registration is declared as confident registration. Otherwise the image matching is not verified and the registration has low confidence. In the above embodiment, the quality of image matching is measured in terms of the posterior probability that image matching is correct given the features extracted as shown in eqn. (4). If the quality of image matching is over a threshold (i.e., $p_{min}$), the image matching is verified. After further derivation, the decision process according to one embodiment of the present invention simply becomes counting the number of inlier, $n_i$, and comparing the result with a threshold. While the quality of image matching can be measured by counting the number of inlier, the quality of image matching can be measured by counting the number of outlier. In this case, if the number of outlier is less than a second threshold, the image matching is verified. Otherwise, image matching is not verified.

In another embodiment, the system uses non-feature based direct matching and calculates the sum of squared distance (SSD) as the measure of quality of image matching. The SSD between images A and B is defined as:

$$D_{SSD}(A, B | T) = \sum_{(x,y)} (A(x,y) - B(T(x,y)))^2, \quad (7)$$

where (*x,y*) is the pixel in the overlap area, *T* is the transformation from image A to B. By carefully choosing a threshold *Dmax,* if *Dssd(A,B|T)<Dmax,* the image matching can be verified and the registration has high confidence. Otherwise the registration is not verified and the registration is not confident. In another embodiment normalized cross correlation (NCC) or mutual information (MI) can be used as a criterion to evaluate the quality of matching and compute the confidence score.

In another embodiment, in order to stitch two sequential images, each image of the pair will be down-sampled to create image pyramids first. From the coarse level, a global transformation will be estimated using exhaustive search within a pre-defined range. The resulting global transformation will be refined in the next level until the final level, which is the original image. After the global transformation is estimated, a free-form deformation transformation can be applied to the overlapped area to estimate the local deformation. The output of the optimization object function can be used as a criterion to evaluate the quality of matching and compute the confidence score.

In another embodiment, in order to stitch two sequential images, each image of the pair will be down-sampled to create image pyramids first. From the coarse level, a global transformation will be estimated by averaging the local transformation, which is computed by applying free-form deformation to the entire image. The resulting global transformation will be refined in the next level until the final level, which is the original image. Such a procedure will be iterated until the process converges to eliminate outlier effect. After the global transformation is estimated, a free-form deformation transformation can be applied to the overlapped area to estimate the local deformation. The output of the optimization object function can be used as a criterion to evaluate the quality of matching and compute the confidence score.

In another embodiment, more than two images can be stitched together with high confidence if and only if the following condition is true. Given the set of images $i1, i2,... ,iN$, for each image $ij$ ($j$=1,2,...$N$), we can find at least one image from the rest of images to match ij with high confidence. There might be multiple images matching $ij$ with high confidence. Otherwise, it means $ij$ cannot be stitched with the rest of images and will be removed from this image set. Above process can be repeated until no image will be removed from the image set. Then all the images in this set can be stitched together to form a large composite image. All the removed images will be displayed individually.

In one embodiment example, $i1, i2,... iN$ are a sequence of images along time domain, where $i1, i2, i3, i5, i6, i7, i8, i12$ are found to find match with high confidence and are stitched together and displayed as composite image $I1$, while $i4, i9, i10$ and $i11$ could not and are displayed as single images. If $i4$ and $i9$ and $i11$ could find match with confidence while $i10$ could not, then $i4, i9$ and $i11$ are stitched together as a composite image 12 and displayed as such while $i10$ is displayed as single image in the video separately.

Sometimes the advantage of stitching too few images and displaying them in one composite image is outweighed by the disadvantage. For example the stitched images have arbitrary size while single image is fixed in dimensions and aspect ratio so looking at two stitched images in a composite image may not be as efficient in time compared with reading these two images in a video displayed at certain frame rate. A threshold T may be chosen to set the minimum number of images matched with high confidence before they are stitched and displayed as a composite image.

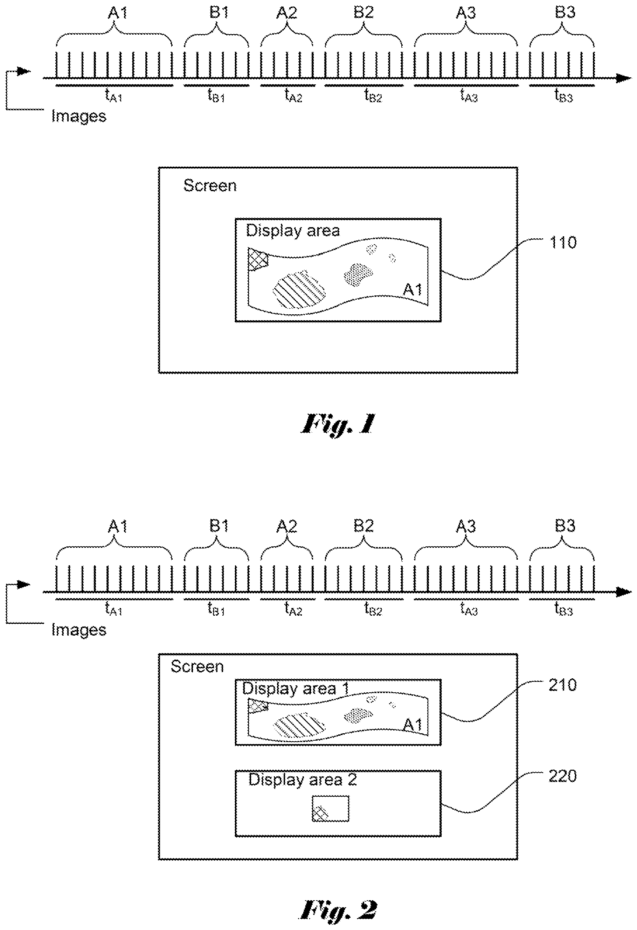

The quality of image matching disclosed above can be used to guide image stitching. When the quality of image matching is high, the registration can be declared to be confident. In one embodiment, the images are stitched to form a larger composite picture for images with high confidence even if there are discontinuities along transit time. For those images declared to be low confidence, the images are not stitched. The un-stitched images are treated as individual images or an image sequence and viewed as video. Fig. 1 illustrates one embodiment according to the present invention, where A1, A2 and A3 represent three groups of images with high confidence throughout the video. Each of A1, A2 and A3 corresponds to images in respective time periods $t_{A1}$, $t_{A2}$ and $t_{A3}$ having high confidence. The images within each group (i.e., A1, A2 or A3) are stitched into one or more larger composite pictures. B1, B2 and B3 correspond to images in respective time periods $t_{B1}$, $t_{B2}$ and $t_{B3}$ having low confidence. In one embodiment, images associated with A1, A2, A3 can be displayed in display area 110, and then then followed by images associated with B1, B2 and B3. Fig. 1 illustrates an instance that composite picture corresponding to group A1 is being displayed. The display order can be A1, A2 and A3 and then followed by B1, B2 and B3. The display may also follow the order of A1, B1, A2, B2, A3 and B3. When images associated with A1, A2, A3 are displayed, a stitched larger image or images can be used to allow a view to examine multiple images at the same time. When images associated with B1, B2 and B3 are displayed, the images will be treated as individual images and they can be displayed one by one manually or displayed as a video sequence at a desirable playback rate. The images are taken at uniform speed in Fig. 1. In another embodiment, images could be taken at non-uniform frame rate.

Fig. 2 illustrates another embodiment according to the present invention. Again, A1, A2 and A3 represent images with high confidence throughout the video. B1, B2 and B3 correspond to images having low confidence. There are two display areas, one is used to display A1, A2 and A3, the other one is used to display B1, B2 and B3. Two display areas (210 and 220) are used to display A1/A2/A3 and B1/B2/B3 separately. Images associated with A1, A2, A3 can be displayed as stitched larger composite in display area 210. Images associated with B1, B2 and B3 can be displayed as individual images. They can be displayed one by one manually or displayed in display area 220 as a video at a desirable play back rate.

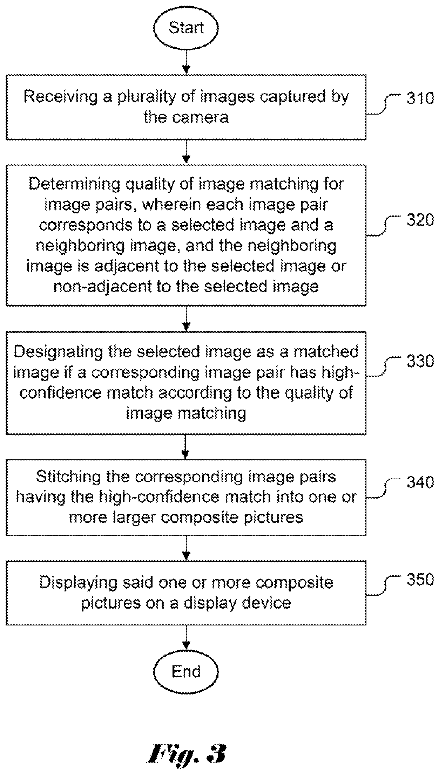

Fig. 3 illustrates an exemplary flowchart of a system for displaying images incorporating image stitching guided by quality of image matching according to an embodiment of the present invention. A plurality of images captured by the camera is received as shown in step 310. The images may be retrieved from memory or received from a processor. The quality of image matching for each image is determined as shown in step 320. The quality of image matching is measured between two images to be matched. The image pair is then designated as a high-confidence matched image pair or a low-confidence unmatched image pair according to the quality of image matching as shown in step 330. The high-confidence images are stitched into one or more larger composite pictures as shown in step 340. The stitched larger composite pictures are displayed on a display device as shown in step 350.

The described examples are to be considered in all respects only as illustrative and not restrictive. Therefore, the scope of the invention is indicated by the appended claims rather than by the foregoing description. All changes which come within the meaning and range of equivalency of the claims are to be embraced within their scope.

**Claims**

1. A method of displaying images of human gastrointestinal (GI) tract captured using a capsule camera travelling through the GI tract, the method comprising:

   receiving a plurality of images captured by the capsule camera (310); and
   the method being **characterized by** comprising:

   determining quality of image matching for image pairs as either high-confidence match or low-confidence match, wherein each image pair corresponds to a selected image and a neighboring image, and the neighboring image is adjacent to the selected image or non-adjacent to the selected image (320);
   designating the selected image as a matched image if a corresponding image pair has high-confidence match according to the quality of image matching (330);
   designating the selected image as an unmatched image if all corresponding image pairs associated with the selected image have low-confidence match according to the quality of image matching, wherein the unmatched images are displayed on a display device as individual images or as a sequence without stitching;
   stitching the corresponding image pairs having the high-confidence match into one or more larger composite pictures (340); and
   displaying said one or more composite pictures on the display device (350).

2. The method of Claim 1, wherein said one or more composite pictures and the unmatched images are displayed on the display device in an interleaved manner, wherein said one or more composite pictures are displayed during first periods and the unmatched images are displayed during second periods, and the first periods and the second periods are non-overlapping, or wherein said one or more composite pictures are displayed in a first display area on the display device and the unmatched images are displayed in a second display area on the display device.

3. The method of Claim 1, wherein the quality of image matching is based on features extracted between each image pair.

4. The method of Claim 3, wherein the quality of image matching is based on posterior probability corresponding to correct image matching under a condition of being provided the features extracted, wherein each feature is modelled as a binary random variable being an inlier or an outlier.

5. The method of Claim 4, wherein the quality of image matching is measured by counting a number of the features belonging to the inliers, and the selected image is designated as the matched image if the number of the features belonging to the inliers exceeds a threshold and the selected image is designated as the unmatched image if the number of the features belonging to the inliers is below the threshold for all image pairs associated with the selected image.

6. The method of Claim 5, wherein the threshold is dependent on a first probability corresponding to the feature being the inlier and a second probability corresponding to the feature being the outlier.

7. The method of Claim 1, wherein the quality of image matching is based on a sum of squared distance (SSD), normalized cross correlation (NCC) or mutual information (MI) between each image pair.

8. The method of Claim 7, wherein the selected image is designated as the matched image if the SSD is smaller than a threshold and the selected image is designated as the unmatched image if the SSD exceeds the threshold for all image pairs associated with the selected image, or wherein the selected image is designated as the matched image if the NCC or MI exceeds a threshold and the selected image is designated as the unmatched image if the NCC or MI is smaller than the threshold for all image pairs associated with the selected image.

9. The method of Claim 1, wherein said determining the quality of image matching for the image pairs comprises:

   generating image pyramids for each image of the corresponding image pairs;
   estimating global transformation at a coarser level of the image pyramids by applying exhaustively search on coarser images of the image pyramids;
   refining the global transformation at a finer level by using a global transformation result at the coarser level;
   applying a local transformation based on free-form deformation to an overlapped area of the image pair to estimate local deformation, wherein parameters of the free form deformation are determined by optimizing an object function of the parameters; and

providing an output from the object function optimized as the quality of image matching for the image pairs.

10. The method of Claim 1, wherein said determining the quality of image matching for the image pairs comprises:

generating image pyramids for each image of the corresponding image pairs;
applying local transformation computed by applying free-form deformation to each entire image pairs, wherein parameters of the free form deformation are determined by optimizing an object function of the parameters;
estimating global transformation by averaging the local transformation on coarser images of the image pyramids;
refining the global transformation at a finer level by using a global transformation result at a coarser level;
applying the free-form deformation to an overlapped area of the image pair to estimate local deformation after the global transformation is estimated at a final level of the image pyramids; and
providing an output from an object function optimized as the quality of image matching for the image pairs.

11. The method of Claim 1, wherein said stitching the corresponding image pairs having the high-confidence match into one or more larger composite pictures is performed only if a number of corresponding image pair for one larger composite picture is greater than a threshold.

12. A system of displaying images of human gastrointestinal (GI) tract captured using a capsule camera travelling through the GI tract, the system comprising:

a display device; and
a processor coupled to the display device, wherein the processor is configured to:

receive a plurality of images captured by the capsule camera (310);
the processor being **characterized by** being configured to:

determine quality of image matching for image pairs as either high-confidence match or low-confidence match, wherein each image pair corresponds to a selected image and a neighboring image, and the neighboring image is adjacent to the selected image or non-adjacent to the selected image (320);
designate the selected image as a matched image if a corresponding image pair has high-confidence match according to the quality of image matching (330);
designate the selected image as an unmatched image if all corresponding image pairs associated with the selected image have low-confidence match according to the quality of image matching, wherein the unmatched images are displayed on the display device as individual images or as a sequence without stitching;
stitch the corresponding image pairs having the high-confidence match into one or more larger composite pictures (320); and
display said one or more composite pictures on the display device (350).

13. The system of Claim 12, wherein said one or more composite pictures and the unmatched images are displayed on the display device in an interleaved manner, wherein said one or more composite pictures are displayed during first periods and the unmatched images are displayed during second periods, and the first periods and the second periods are non-overlapping, or wherein said one or more composite pictures are displayed in a first display area on the display device and the unmatched images are displayed in a second display area on the display device.

14. The system of Claim 12, wherein the quality of image matching is based on features extracted between each image pair, or wherein the quality of image matching is based on a sum of squared distance (SSD), normalized cross correlation (NCC) or mutual information (MI) between each image pair.

**Patentansprüche**

1. Verfahren zum Anzeigen von Bildern eines menschlichen gastrointestinalen (GI) Trakts, die unter Verwendung einer Kapselkamera aufgenommen werden, die sich durch den GI-Trakt fortbewegt, wobei das Verfahren umfasst:

Empfangen (310) von einer Mehrzahl von Bildern, die von der Kapselkamera aufgenommen werden; und

**dadurch gekennzeichnet, dass** das Verfahren umfasst:

Bestimmen (320) einer Qualität eines Bildabgleichs für Bildpaare als entweder ein Abgleich mit hoher Konfidenz oder ein Abgleich mit niedriger Konfidenz, wobei jedes Bildpaar einem ausgewählten Bild und einem benachbarten Bild entspricht, und wobei das benachbarte Bild angrenzend zu dem ausgewählten Bild oder nicht angrenzend zu dem ausgewählten Bild ist;

Kennzeichnen (330) des ausgewählten Bilds als ein abgeglichenes Bild, wenn ein entsprechendes Bildpaar einen Abgleich mit hoher Konfidenz gemäß der Qualität des Bildabgleichs aufweist;

Kennzeichnen des ausgewählten Bilds als ein nicht abgeglichenes Bild, wenn alle entsprechenden Bildpaare, die mit dem ausgewählten Bild assoziiert werden, einen Abgleich mit niedriger Konfidenz gemäß der Qualität des Bildabgleichs aufweisen, wobei die nicht abgeglichenen Bilder auf einer Anzeigevorrichtung als individuelle Bilder oder als eine Sequenz ohne Aneinanderfügung angezeigt werden;

Aneinanderfügen (340) der entsprechenden Bildpaare, die den Abgleich mit hoher Konfidenz aufweisen, in ein oder mehrere größere zusammengesetzte Bilder; und

Anzeigen (350) des einen oder der mehreren zusammengesetzten Bilder auf der Anzeigevorrichtung.

2.  Verfahren nach Anspruch 1, wobei das eine oder die mehreren zusammengesetzten Bilder und die nicht abgeglichenen Bilder auf der Anzeigevorrichtung in einer verschachtelten Weise angezeigt werden, wobei das eine oder die mehreren zusammengesetzten Bilder während ersten Zeiträumen angezeigt werden und die nicht abgeglichenen Bilder während zweiten Zeiträumen angezeigt werden, und wobei die ersten Zeiträume und die zweiten Zeiträume nicht überlappend sind, oder wobei das eine oder die mehreren zusammengesetzten Bilder in einem ersten Anzeigegebiet auf der Anzeigevorrichtung angezeigt werden und die nicht abgeglichenen Bilder in einem zweiten Anzeigegebiet auf der Anzeigevorrichtung angezeigt werden.

3.  Verfahren nach Anspruch 1, wobei die Qualität des Bildabgleichs auf Merkmalen basiert, die zwischen jedem Bildpaar extrahiert werden.

4.  Verfahren nach Anspruch 3, wobei die Qualität des Bildabgleichs auf einer Nachfolgewahrscheinlichkeit basiert, die einem korrekten Bildabgleich unter einer Bedingung entspricht, dass die extrahierten Merkmale bereitgestellt werden, wobei jedes Merkmal als eine binäre Zufallsvariable modelliert ist, die ein Erwartungswert oder ein Ausreißer ist.

5.  Verfahren nach Anspruch 4, wobei die Qualität des Bildabgleichs durch ein Zählen einer Anzahl der Merkmale gemessen wird, die den Erwartungswerten zugehören, und wobei das ausgewählte Bild als das abgeglichene Bild gekennzeichnet wird, wenn die Anzahl der Merkmale, die den Erwartungswerten zugehören, einen Schwellenwert übersteigt, und wobei das ausgewählte Bild als das nicht abgeglichene Bild gekennzeichnet wird, wenn die Anzahl der Merkmale, die den Erwartungswerten zugehören, unterhalb des Schwellenwerts ist, für alle Bildpaare, die mit dem ausgewählten Bild assoziiert werden.

6.  Verfahren nach Anspruch 5, wobei der Schwellenwert abhängig ist von einer ersten Wahrscheinlichkeit, die dem Merkmal entspricht, das der Erwartungswert ist, und von einer zweiten Wahrscheinlichkeit, die dem Merkmal entspricht, das der Ausreißer ist.

7.  Verfahren nach Anspruch 1, wobei die Qualität des Bildabgleichs auf einer Summe der quadratischen Distanz (SSD), einer normalisierten Kreuzkorrelation (NCC) oder auf wechselseitigen Informationen (MI) zwischen jedem Bildpaar basiert.

8.  Verfahren nach Anspruch 7, wobei das ausgewählte Bild als das abgeglichene Bild gekennzeichnet wird, wenn die SSD kleiner ist als ein Schwellenwert, und das ausgewählte Bild als das nicht abgeglichene Bild gekennzeichnet wird, wenn die SSD den Schwellenwert für alle Bildpaare übersteigt, die mit dem ausgewählten Bild assoziiert werden, oder wobei das ausgewählte Bild als das abgeglichene Bild gekennzeichnet wird, wenn die NCC oder MI einen Schwellenwert übersteigt, und das ausgewählte Bild als das nicht abgeglichene Bild gekennzeichnet wird, wenn die NCC oder MI kleiner ist als der Schwellenwert für alle Bildpaare, die mit dem ausgewählten Bild assoziiert werden.

9.  Verfahren nach Anspruch 1, wobei das Bestimmen der Qualität des Bildabgleichs für die Bildpaare umfasst:

Erzeugen von Bildpyramiden für jedes Bild der entsprechenden Bildpaare;

Schätzen einer globalen Transformation bei einem gröberen Niveau der Bildpyramiden durch ein Anwenden einer umfassenden Suche nach gröberen Bildern der Bildpyramiden;
Verfeinern der globalen Transformation bei einem feineren Niveau unter Verwendung eines Resultats der globalen Transformation bei dem gröberen Niveau;
Anwenden einer lokalen Transformation basierend auf einer freiförmigen Deformation auf ein überlappendes Gebiet des Bildpaars zum Schätzen einer lokalen Deformation, wobei Parameter der freiförmigen Deformation bestimmt werden durch ein Optimieren einer Objektfunktion der Parameter; und
Bereitstellen einer Ausgabe von der Objektfunktion, die hinsichtlich der Qualität des Bildabgleichs für die Bildpaare optimiert ist.

10. Verfahren nach Anspruch 1, wobei das Bestimmen der Qualität des Bildabgleichs für die Bildpaare umfasst:

Erzeugen von Bildpyramiden für jedes Bild der entsprechenden Bildpaare;
Anwenden einer lokalen Transformation, die berechnet wird durch ein Anwenden einer freiförmigen Deformation auf jedes gesamte Bildpaar, wobei Parameter der freiförmigen Deformation bestimmt werden durch ein Optimieren einer Objektfunktion der Parameter;
Schätzen einer globalen Transformation durch Mitteln der lokalen Transformation auf gröbere Bilder der Bildpyramiden;
Verfeinern der globalen Transformation bei einem feineren Niveau unter Verwendung eines Resultats der globalen Transformation bei einem gröberen Niveau;
Anwenden der freiförmigen Deformation auf ein überlappendes Gebiet des Bildpaares zum Schätzen einer lokalen Deformation, nachdem die globale Transformation bei einem feineren Niveau der Bildpyramiden geschätzt wird; und
Bereitstellen einer Ausgabe von einer Objektfunktion, die hinsichtlich der Qualität des Bildabgleichs für die Bildpaare optimiert ist.

11. Verfahren nach Anspruch 1, wobei das Aneinanderfügen der entsprechenden Bildpaare, die den Abgleich mit hoher Konfidenz aufweisen, in ein oder mehrere größere zusammengesetzte Bilder nur durchgeführt wird, wenn eine Anzahl an entsprechenden Bildpaaren für ein größeres zusammengesetztes Bild größer ist als ein Schwellenwert.

12. System zum Anzeigen von Bildern eines menschlichen gastrointestinalen (GI) Trakts, die unter Verwendung einer Kapselkamera aufgenommen werden, die sich durch den GI-Trakt fortbewegt, wobei das System umfasst:

eine Anzeigevorrichtung; und
ein Prozessor, der mit der Anzeigevorrichtung gekoppelt ist, wobei der Prozessor konfiguriert ist zum:

Empfangen (310) einer Mehrzahl von Bildern, die durch die Kapselkamera aufgenommen werden;
**dadurch gekennzeichnet, dass** der Prozessor konfiguriert ist zum:

Bestimmen (320) einer Qualität eines Bildabgleichs für Bildpaare als entweder ein Abgleich mit hoher Konfidenz oder ein Abgleich mit niedriger Konfidenz, wobei jedes Bildpaar einem ausgewählten Bild und einem benachbarten Bild entspricht, und wobei das benachbarte Bild angrenzend zu dem ausgewählten Bild oder nicht angrenzend zu dem ausgewählten Bild ist;
Kennzeichnen (330) des ausgewählten Bilds als ein abgeglichenes Bild, wenn ein entsprechendes Bildpaar einen Abgleich mit hoher Konfidenz gemäß der Qualität des Bildabgleichs aufweist;
Kennzeichnen des ausgewählten Bilds als ein nicht abgeglichenes Bild, wenn alle entsprechenden Bildpaare, die mit dem ausgewählten Bild assoziiert werden, einen Abgleich mit niedriger Konfidenz gemäß der Qualität des Bildabgleichs aufweisen, wobei die nicht abgeglichenen Bilder auf der Anzeigevorrichtung als individuelle Bilder oder als eine Sequenz ohne Aneinanderfügung angezeigt werden;
Aneinanderfügen (320) der entsprechenden Bildpaare, die den Abgleich mit hoher Konfidenz aufweisen, in ein oder mehrere größere zusammengesetzte Bilder; und
Anzeigen (350) des einen oder der mehreren zusammengesetzten Bilder auf der Anzeigevorrichtung.

13. System nach Anspruch 12, wobei das eine oder die mehreren zusammengesetzten Bilder und die nicht abgeglichenen Bilder auf der Anzeigevorrichtung in einer verschachtelten Weise angezeigt werden, wobei das eine oder die mehreren zusammengesetzten Bilder während ersten Zeiträumen angezeigt werden und die nicht angeglichenen Bilder während zweiten Zeiträumen angezeigt werden, und wobei die ersten Zeiträume und die zweiten Zeiträume nicht überlappend sind, oder wobei das eine oder die mehreren zusammengesetzten Bilder in einem ersten Anzeigegebiet auf der Anzeigevorrichtung angezeigt werden und die nicht abgeglichenen Bilder in einem zweiten Anzeigegegebiet auf der Anzeigevorrichtung angezeigt werden.

14. System nach Anspruch 12, wobei die Qualität des Bildabgleichs auf Merkmalen basiert, die zwischen jedem Bildpaar extrahiert werden, oder wobei die Qualität des Bildabgleichs auf einer Summe der quadratischen Distanz (SSD), auf einer normalisierten Kreuzkorrelation (NCC) oder auf wechselseitigen Informationen (MI) zwischen jedem Bildpaar basiert.

**Revendications**

1. Procédé d'affichage d'images du tractus gastrointestinal humain (GI) capturées en utilisant une vidéocapsule se déplaçant dans le tractus GI, le procédé comprenant l'étape consistant à :

   recevoir une pluralité d'images capturées par la vidéocapsule (310) ; et
   le procédé étant **caractérisé en ce qu'**il comprend les étapes consistant à :

   déterminer la qualité de concordance d'image pour des paires d'images comme étant soit une concordance à confiance élevée, soit une concordance à faible confiance, où chaque paire d'images correspond à une image sélectionnée et à une image voisine, et l'image voisine est adjacente à l'image sélectionnée ou non adjacente à l'image sélectionnée (320) ;
   désigner l'image sélectionnée comme image concordante si une paire d'images correspondante présente une concordance à confiance élevée selon la qualité de concordance d'image (330) ;
   désigner l'image sélectionnée comme image non concordante si toutes les paires d'images correspondantes associées à l'image sélectionnée présentent une concordance à faible confiance selon la qualité de concordance d'image, où les images non concordantes sont affichées sur un dispositif d'affichage sous forme d'images individuelles ou sous forme d'une séquence sans assemblage ;
   assembler les paires d'images correspondantes présentant la concordance à confiance élevée en une ou plusieurs photos composites (340) plus grandes ; et
   afficher lesdites une ou plusieurs photos composites sur le dispositif d'affichage (350).

2. Procédé selon la revendication 1, dans lequel lesdites une ou plusieurs photos composites et les images non concordantes sont affichées sur le dispositif d'affichage d'une manière entrelacée, dans lequel lesdites une ou plusieurs photos composites sont affichées pendant des premières périodes et les images non concordantes sont affichées pendant des deuxièmes périodes, et les premières périodes et les deuxièmes périodes ne se chevauchent pas, ou dans lequel lesdites une ou plusieurs photos composites sont affichées dans une première zone d'affichage sur le dispositif d'affichage et les images non concordantes sont affichées dans une deuxième zone d'affichage sur le dispositif d'affichage.

3. Procédé selon la revendication 1, dans lequel la qualité de concordance d'image est basée sur des caractéristiques extraites entre chaque paire d'images.

4. Procédé selon la revendication 3, dans lequel la qualité de concordance d'image est basée sur une probabilité postérieure correspondant à une concordance d'image correcte à condition que les caractéristiques extraites lui soient fournies, où chaque caractéristique est modélisée comme une variable aléatoire binaire qui est une valeur normale, inlier, ou une valeur aberrante, outlier.

5. Procédé selon la revendication 4, dans lequel la qualité de concordance d'image est mesurée en comptant un nombre des caractéristiques appartenant aux inliers, et l'image sélectionnée est désignée comme l'image concordante si le nombre des caractéristiques appartenant aux inliers dépasse un seuil, et l'image sélectionnée est désignée comme l'image non concordante si le nombre des caractéristiques appartenant aux inliers est en dessous du seuil pour toutes les paires d'images associées à l'image sélectionnée.

6. Procédé selon la revendication 5, dans lequel le seuil dépend d'une première probabilité correspondant à la caractéristique qui est l'inlier, et d'une deuxième probabilité correspondant à la caractéristique qui est l'outlier.

7. Procédé selon la revendication 1, dans lequel la qualité de concordance d'image est basée sur une somme de distance au carré (SSD), une corrélation croisée normalisée (NCC) ou une information mutuelle (MI) entre chaque paire d'images.

8. Procédé selon la revendication 7, dans lequel l'image sélectionnée est désignée comme l'image concordante si la SSD est inférieure à un seuil, et l'image sélectionnée est désignée comme l'image non concordante si la SSD dépasse le seuil pour toutes les paires d'images associées à l'image sélectionnée, ou dans lequel l'image sélectionnée est désignée comme l'image concordante si la NCC ou la MI dépasse un seuil, et l'image sélectionnée est désignée comme l'image non concordante si la NCC ou la MI est inférieure au seuil pour toutes les paires d'images associées à l'image sélectionnée.

9. Procédé selon la revendication 1, dans lequel ladite détermination de la qualité de concordance d'image pour les paires d'images comprend les étapes consistant à :

générer des pyramides d'images pour chaque image des paires d'images correspondantes ;
estimer une transformation globale à un niveau plus grossier des pyramides d'images en appliquant une recherche exhaustive sur des images plus grossières des pyramides d'images ;
affiner la transformation globale à un niveau plus fin en utilisant un résultat de transformation globale au niveau plus grossier ;
appliquer une transformation locale sur la base d'une déformation de forme libre à une zone de chevauchement de la paire d'images pour estimer la déformation locale, où des paramètres de la déformation de forme libre sont déterminés en optimisant une fonction objet des paramètres ; et
fournir une sortie de la fonction objet optimisée comme la qualité de concordance d'image pour les paires d'images.

10. Procédé selon la revendication 1, dans lequel ladite détermination de la qualité de concordance d'image pour les paires d'images comprend les étapes consistant à :

générer des pyramides d'images pour chaque image des paires d'images correspondantes ;
appliquer une transformation locale calculée en appliquant une déformation de forme libre à chaque paire d'images entière, où des paramètres de la déformation de forme libre sont déterminés en optimisant une fonction objet des paramètres ;
estimer une transformation globale en moyennant la transformation locale sur des images plus grossières des pyramides d'images ;
affiner la transformation globale à un niveau plus fin en utilisant un résultat de transformation globale à un niveau plus grossier ;
appliquer la déformation de forme libre à une zone de chevauchement de la paire d'images pour estimer la déformation locale après que la transformation globale a été estimée à un niveau final des pyramides d'images ; et
fournir une sortie d'une fonction objet optimisée comme la qualité de concordance d'image pour les paires d'images.

11. Procédé selon la revendication 1, dans lequel ledit assemblage des paires d'images correspondantes présentant la concordance à confiance élevée en une ou plusieurs photos composites plus grandes est effectué seulement si un nombre de paires d'images correspondantes pour une image composite plus grande est supérieur à un seuil.

12. Système d'affichage d'images de tractus gastrointestinal humain (GI) capturé en utilisant une vidéocapsule se déplaçant dans le tractus GI, le système comprenant :

un dispositif d'affichage ; et
un processeur couplé au dispositif d'affichage, où le processeur est configuré pour :

recevoir une pluralité d'images capturées par la vidéocapsule (310) ;
le processeur étant **caractérisé en ce qu'**il est configuré pour :

déterminer la qualité de concordance d'image pour des paires d'images comme étant soit une concordance à confiance élevée, soit une concordance à faible confiance, où chaque paire d'images correspond à une image sélectionnée et à une image voisine, et l'image voisine est adjacente à l'image sélectionnée ou non adjacente à l'image sélectionnée (320) ;
désigner l'image sélectionnée comme image concordante si une paire d'images correspondante présente une concordance à confiance élevée selon la qualité de concordance d'image (330) ;

désigner l'image sélectionnée comme image non concordante si toutes les paires d'images correspondantes associées à l'image sélectionnée présentent une concordance à faible confiance selon la qualité de concordance d'image, où les images non concordantes sont affichées sur le dispositif d'affichage sous forme d'images individuelles ou sous forme d'une séquence sans assemblage ;

assembler les paires d'images correspondantes présentant la concordance à confiance élevée en une ou plusieurs photos composites plus grandes (320) ; et

afficher lesdites une ou plusieurs photos composites sur le dispositif d'affichage (350).

**13.** Système selon la revendication 12, dans lequel lesdites une ou plusieurs photos composites et les images non concordantes sont affichées sur le dispositif d'affichage d'une manière entrelacée, dans lequel lesdites une ou plusieurs photos composites sont affichées pendant des premières périodes et les images non concordantes sont affichées pendant des deuxièmes périodes, et les premières périodes et les deuxièmes périodes ne se chevauchent pas, ou dans lequel lesdites une ou plusieurs photos composites sont affichées dans une première zone d'affichage sur le dispositif d'affichage et les images non concordantes sont affichées dans une deuxième zone d'affichage sur le dispositif d'affichage.

**14.** Système selon la revendication 12, dans lequel la qualité de concordance d'image est basée sur des caractéristiques extraites entre chaque paire d'images, ou dans lequel la qualité de concordance d'image est basée sur une somme de distance au carré (SSD), une corrélation croisée normalisée (NCC) ou une information mutuelle (MI) entre chaque paire d'images.

A1    B1    A2    B2    A3    B3

$t_{A1}$    $t_{B1}$    $t_{A2}$    $t_{B2}$    $t_{A3}$    $t_{B3}$

Images

Screen

Display area

A1

— 110

*Fig. 1*

A1    B1    A2    B2    A3    B3

$t_{A1}$    $t_{B1}$    $t_{A2}$    $t_{B2}$    $t_{A3}$    $t_{B3}$

Images

Screen

Display area 1

A1

— 210

Display area 2

— 220

*Fig. 2*

Start

Receiving a plurality of images captured by the camera — 310

Determining quality of image matching for image pairs, wherein each image pair corresponds to a selected image and a neighboring image, and the neighboring image is adjacent to the selected image or non-adjacent to the selected image — 320

Designating the selected image as a matched image if a corresponding image pair has high-confidence match according to the quality of image matching — 330

Stitching the corresponding image pairs having the high-confidence match into one or more larger composite pictures — 340

Displaying said one or more composite pictures on a display device — 350

End

*Fig. 3*

## REFERENCES CITED IN THE DESCRIPTION

**Patent documents cited in the description**

- US 64227506
- US 2009074265 A1
- US 2010149183 A1

**Non-patent literature cited in the description**

- **SZELISKI.** Image Alignment and Stitching: A Tutorial. *Microsoft Research Technical Report MSR-TR-2004-92,* 10 December 2006